# EUROPÄISCHE PATENTSCHRIFT

(11) **EP 2 251 408 B1**
(45) Veröffentlichungstag und Bekanntmachung des Hinweises auf die Patenterteilung: **27.06.2012**
(21) Anmeldenummer: 09006326.4
(22) Anmeldetag: 11.05.2009
(51) Int. Cl.: C12M 1/107, C12M 1/04

(54) **Verfahren und Vorrichtung zum Betreiben einer Fermentationsanlage**
Method and device for operating a fermentation assembly
Procédé et dispositif destinés au fonctionnement d'une installation de fermentation

(43) Veröffentlichungstag der Anmeldung: 17.11.2010
(60) Teilanmeldung: 11005576.1
(73) Patentinhaber: KOMPOFERM GmbH, 33428 Marienfeld (DE)
(72) Erfinder: Eggersmann, Karlgünter, 33428 Marienfeld (DE)
(74) Vertreter: Schober, Mirko

(56) Entgegenhaltungen:
- EP-A- 1 428 868
- EP-A- 1 736 535
- EP-A- 1 811 015
- EP-A- 1 997 875
- WO-A-02/06439
- WO-A-2005/054423

## Beschreibung

Die Erfindung betrifft ein Verfahren zum Betreiben einer Fermentationsanlage nach dem Oberbegriff des Anspruchs 1.

Bei entsprechenden Verfahren wird ein Substrat in einem Fermenter mit Perkolat durchströmt, welches zumeist am Boden des Fermenters aufgefangen, in einen Perkolatbehälter zurückgeführt und von dort aus dem Fermenter wieder zugeführt werden kann. Hierdurch entsteht im Fermenter und auch im Perkolatbehälter methanhaltiges Biogas, welches aus dem Fermenter und/oder dem Perkolatbehälter abgeführt und einer Biogasaufbereitung zur Erhöhung der Methankonzentration zugeführt wird. Bei der Biogasaufbereitung wird das zu gewinnende Biogas von weiteren unerwünschten Bestandteilen getrennt und der weiteren Verwendung zugeführt. Bei der Aufbereitung entsteht unter anderem eine erhebliche Menge an CO₂.

Um das Biogas aus dem Fermenter am Ende des Prozesses der Biogasaufbereitung zuzuführen, muss das Biogashaltige Volumen oberhalb des Substrates nach dem Fermentationsprozess mit einem Spülgas gespült werden, um das Biogas aus dem Volumen abzufahren. Aus EP 1 997 875 A1 ist bekannt, hierzu aus der Aufbereitung stammendes CO₂-haltiges Gas einzusetzen.

Während des Fermentationsprozesses entstehen auch nicht unerhebliche Mengen an Biogas, welche allerdings im Substrat "gefangen" sind. Diese werden beim Materialhandling oder der Gärrestnachbearbeitung freigesetzt. Die freigesetzten Biogasanteile, insbesondere Methan, gelangen so unkontrolliert in die Atmosphäre.

Die EP 1 428 868 offenbart eine Fermentationsanlage mit wenigstens einem Fermenter, welcher Biogasanschlüsse oberhalb der Biomasse und unterhalb eines die Biomasse tragenden Belüftungsbodens aufweist. Dadurch wird eine Durchströmung der Biomasse mit Biogas ermöglicht.

Der Erfindung liegt daher die Aufgabe zugrunde, ein Verfahren anzugeben, bei dem die erwähnten Nachteile nicht auftreten und der Methanschlupf auf ein Minimum reduziert werden kann.

Erfindungsgemäß wird diese Aufgabe mit den Merkmalen des Anspruchs 1 gelöst. Vorteilhafte Ausführungsformen finden sich in den Unteransprüchen.

Beim erfindungsgemäßen Verfahren wird das CO₂-haltige Spülgas durch das Substrat geleitet. Bevorzugt wird unter hohem Druck das Spülgas im Bodenbereich möglichst flächig in den Fermenter eingeleitet. So wird das Substrat durchspült und darin vorhandene Biogasanteile gelangen insbesondere in das Volumen oberhalb des Substrates und können in bekannter Weise zur Biogasaufbereitung abgefahren werden. Das CO₂-haltige Spülgas wird dabei als Abgas aus einer Biogasaufbereitung entnommen.

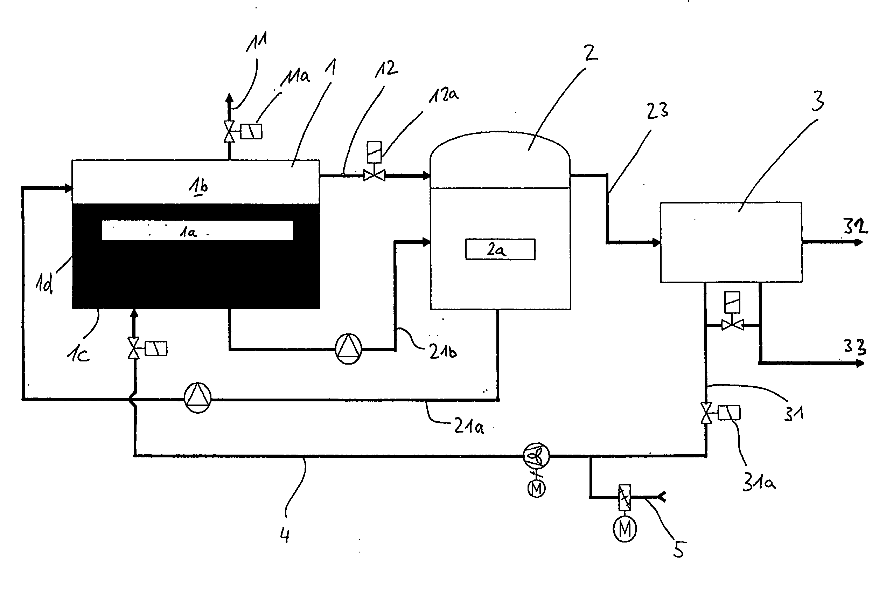

Die Erfindung wird nachfolgend anhand des in der Figur gezeigten Ausführungsbeispiels schematisch näher erläutert.

Ein Fermenter 1 wird mit einem Substrat 1a beschickt. Gezeigt ist lediglich ein Fermenter, jedoch sind Anlagen mit einer Mehrzahl Fermenter möglich. Der Fermenter 1 ist mit einem Perkolatbehälter 2 verbunden durch eine Zuführungsleitung 21a, mit der Perkolat 2a in den Fermenter 1 eingebracht wird und während des Fermentationsprozesses durch das Substrat 1a sickert. Das Perkolat wird im unteren Bereich des Fermenters 1 aufgefangen und über eine Rücklaufleitung 21b wieder dem Perkolatbehälter 2 zugeführt.

Beim Fermentationsprozess entsteht Biogas, welches sich im Fermenter 1 vorrangig im Volumen 1b über dem Substrat 1a sammelt. Zudem befindet sich auch Biogas im Substrat 1a, welches darin eingeschlossen ist und nicht ohne weiteres in das Volumen 1b gelangen kann. Des Weiteren entsteht auch im Perkolatbehälter 2 zusätzliches Biogas. Das im Fermenter in den Bereichen 1b und 1a vorhandene Biogas wird durch den erfindungsgemäßen Spülvorgang abgefahren. Hierzu ist zum einen eine Biogasleitung 12 zwischen Fermenter 1 und Perkolatbehälter 2 und eine weitere Leitung 23 zwischen dem Perkolatbehälter 2 und einer Aufbereitungseinrichtung 3 vorgesehen. Zum Spülen wird das in der Aufbereitungseinrichtung 3 bei der Aufbereitung entstehende CO₂-haltige Gas verwendet. Im Folgenden wird unter CO₂-haltigem Gas ein Gas verstanden, welches überwiegend CO₂ enthält. Es kann auch ausschließlich CO₂ enthalten, andere Bestandteile sind aber auch denkbar.

Bei der Biogasaufbereitung entsteht neben hochkonzentriertem Methan, welches über die Leitung 32 zur Verfügung gestellt wird, überwiegend CO₂-haltiges Abgas, das über die Leitung 33 abgeführt werden kann, oder über die Leitung 31 in die zum Fermenter 1 führende Spülleitung 4 eingeleitet wird. Der Spülanschluss am Fermenter 1 ist so positioniert, dass das durch die Spülleitung 4 zugeführte Spülgas durch das Substrat hindurch geleitet wird. Bevorzugt ist hierzu im Boden 1c oder Bodenbereich des Fermenters 1 oder an der Seite in einem noch von Substrat 1a bedeckten Bereich 1d des Fermenters 1 der Anschluss für die Spülleitung 4 vorgesehen. Zudem kann eine Verteileinrichtung (nicht gezeigt) vorgesehen sein, um das Spülgas an mehreren Stellen durch das Substrat zu leiten. Dies kann im Fermenter 1 oder bereits durch Aufteilen der Spülleitung 4 vor dem Fermenter 1 geschehen. In jedem Fall kann eine Mehrzahl von Spülanschlüssen vorgesehen werden.

Das CO₂-haltige Spülgas wird unter Druck in den Fermenter 1 eingeleitet, sodass noch im Substrat 1a festgehaltenes Biogas aus dem Substrat 1a verdrängt wird. Dabei wird der Anschluss zur Biogasleitung 12 bzw. ein entsprechendes Ventil 12a geöffnet, sodass das aus dem Substrat 1a und dem substratfreien Volumen 1b verdrängte Biogas über den Perkolatbehälter 2 und die Leitung 23 zur Aufbereitungseinrichtung 2 abgefahren wird. Ist das Biogas aus dem Fermenter 1 abgefahren bzw. ist ein vorgegebener Umschaltpunkt erreicht, der sich beispielsweise aus einem Schwellenwert der Biogas(Methan)-Konzentration ergeben kann, wird das Ventil 12a in der Leitung 12 wieder geschlossen und das Abluftventil 11a zur Abluftleitung 11 geöffnet; das Ventil 31a der CO₂-Leitung wird geschlossen und die CO₂-Zufuhr wird gestoppt. Es erfolgt nun weitere Spülung durch Frischluft, welche aus einer Frischluftleitung 5 der Spülleitung 4 zugeführt wird. Die durch die Abluftleitung 11 aus dem Fermenter entweichende Abluft wird dann einer hier nicht näher beschriebenen Abluftbehandlung und/oder -ableitung unterzogen.

Mit dem erfindungsgemäßen Verfahren kann der Methanschlupf durch das erfindungsgemäße Verfahren deutlich reduziert werden. Zudem kann die Effizienz der Biogasgewinnung gesteigert werden, weil auch das im Substrat noch vorhandene Biogas der Aufbereitung zugeführt werden kann.

## Patentansprüche

1. Verfahren zum Betreiben einer Fermentationsanlage mit wenigstens einem Fermenter (1), der mit einem Substrat (1a) beschickt ist, wobei das Substrat (1a) mit Perkolat (2a) aus einem Perkolatbehälter (2) perkoliert wird und über den Perkolationsprozess im Fermenter (1) oder/ und im Perkolatbehälter (2) entstehendes Biogas abgezogen wird, wobei das abgezogene Biogas in einer Biogasaufbereitungseinrichtung (3) aufbereitet und bei der Aufbereitung entstehendes CO₂-haltiges Abgas mittels einer Spülleitung (4) zum Abfahren des im Fermenter (1) entstandenen Biogases in den Fermenter (1) eingeleitet wird, wobei das CO₂-haltige Spülgas so in den Fermenter (1) eingeleitet wird, dass es durch das Substrat (1a) hindurch geleitet wird.

2. Verfahren nach Anspruch 1,
**dadurch gekennzeichnet,**
**dass** das CO₂-haltige Spülgas so durch das Substrat geleitet wird, dass im Substrat (1a) vorhandenes Gas in das substratfreie Volumen des Fermenters (1) verdrängt wird.

3. Verfahren nach einem der vorherigen Ansprüche,
**dadurch gekennzeichnet,**
**dass** das CO₂-haltige Spülgas in den Boden des Fermenters (1) eingeleitet wird.

4. Verfahren nach Anspruch 3,
**dadurch gekennzeichnet,**
**dass** das CO₂-haltige Spülgas im Bereich des Bodens des Fermenters (1) verteilt wird, sodass das Substrat (1a) im Wesentlichen gleichmäßig von unten nach oben mit dem CO₂-haltigen Spülgas durchströmt wird.

5. Verfahren nach einem der vorherigen Ansprüche,
**dadurch gekennzeichnet,**
**dass** nach dem Spülen mit CO₂-haltigem Spülgas Frischluft über die Spülleitung (4) in den Fermenter (1) eingeleitet wird.

6. Verfahren nach einem der vorherigen Ansprüche,
**dadurch gekennzeichnet,**
**dass** während des Einleitens des CO₂-haltigen Spülgases der Biogasabzug über eine Abzugsleitung (12, 23) erfolgt.

7. Verfahren nach einem der vorherigen Ansprüche,
**dadurch gekennzeichnet,**
**dass** nach Beendigen des Spülvorgangs mit CO₂-haltigem Gas der Biogasabzug gesperrt und eine Abluftleitung (11) geöffnet wird.

## Claims

1. Method for operating a fermentation assembly having at least one fermenter (1) which is filled with a substrate (1a) wherein the substrate (1a) is percolated with percolate (2a) from a percolate container (2) and biogas arising through the percolation process in the fermenter (1) and/or in the percolate container (2) is extracted, wherein the extracted biogas is processed in a biogas processing unit (3) and CO₂-containing waste gas arising during the processing is introduced by means of a flushing pipe (4) into the fermenter in order to discharge the biogas arising in the fermenter (1), wherein the CO₂-containing flushing gas is introduced into the fermenter (1) in such a way that it is directed through the substrate (1a).

2. Method according to claim 1
**characterised in that**
the CO₂-containing flushing gas is directed through the substrate so that gas present in the substrate (1a) is compressed into the substrate-free volume of the fermenter (1).

3. Method according to one of the preceding claims
**characterised in that**
the CO₂-containing flushing gas is directed into the bottom of the fermenter (1).

4. Method according to claim 3
**characterised in that**
the CO₂-containing flushing gas is distributed around the bottom of the fermenter (1) so that the CO₂-containing flushing gas permeates up from the bottom through the substrate (1a) substantially uniformly.

5. Method according to one of the preceding claims
**characterised in that**
after flushing through with the CO₂-containing flushing gas fresh air is introduced into the fermenter (1) via the flushing pipe (4).

6. Method according to one of the preceding claims
**characterised in that**
during the introduction of the CO₂-containing flushing gas the biogas extraction is carried out via a discharge pipe (12, 23).

7. Method according to one of the preceding claims
**characterised in that**
at the end of the flushing process with the CO₂-containing gas the biogas extraction is shut off and a waste air pipe (11) is opened.

## Revendications

1. Procédé destiné au fonctionnement d'une installation de fermentation, avec au moins un fermenteur (1), qui est chargé d'un substrat (1a), sachant que ledit substrat (1a) est percolé avec du percolat (2a) en provenance d'un conteneur de percolat (2) et que le biogaz, généré par percolation dans le fermenteur (1) ou/et dans le conteneur de percolat (2), est prélevé, ledit biogaz prélevé étant traité dans une installation de traitement de biogaz (3), et le gaz contenant du CO₂, généré au cours du traitement étant introduit dans le fermenteur (1), par l'intermédiaire d'une conduite de balayage (4), pour chasser le biogaz généré dans le fermenteur (1), sachant que le gaz de balayage contenant du CO₂ est introduit dans le fermenteur (1) de sorte qu'il passe à travers le substrat (1a).

2. Procédé selon la revendication 1,
**caractérisé en ce que** le gaz de balayage contenant du CO₂ est conduit à travers le substrat de sorte que le gaz, présent dans ledit substrat (1a), soit refoulé dans la partie du fermenteur (1) exempte de substrat.

3. Procédé selon l'une des revendications précédentes,
**caractérisé en ce que** le gaz de balayage contenant du CO₂ est conduit à travers le fond du fermenteur (1).

4. Procédé selon la revendication 3,
**caractérisé en ce que** le gaz de balayage contenant du CO₂ est réparti dans la région du fond du fermenteur (1) de sorte que le substrat (1a) soit traversé sensiblement uniformément, du bas vers le haut, par le flux de gaz de balayage contenant du CO₂.

5. Procédé selon l'une des revendications précédentes,
**caractérisé en ce que** de l'air frais est introduit dans le fermenteur (1), par l'intermédiaire de la conduite de balayage, après le balayage au moyen du gaz de balayage contenant du CO₂.

6. Procédé selon l'une des revendications précédentes,
**caractérisé en ce que**, pendant l'introduction du gaz de balayage contenant du CO₂ le prélèvement du biogaz est effectué, par l'intermédiaire d'une conduite de prélèvement (12, 23).

7. Procédé selon l'une des revendications précédentes,
**caractérisé en ce que** le prélèvement de biogaz est arrêté, et qu'une conduite d'échappement (11) est ouverte, après la fin de l'opération de balayage.
